# EUROPEAN PATENT APPLICATION

(11) **EP 3 222 237 A1**
(43) Date of publication of application: **27.09.2017**
(21) Application number: 17162087.5
(22) Date of filing: 21.03.2017
(51) Int. Cl.: A61B 18/08, A61B 17/00, A61B 18/00

(54) **MEDICAL DEVICE**

(30) Priority: 24.03.2016 US 201662312533 P
(71) Applicant: Cook Medical Technologies LLC, Bloomington, IN 47404 (US)
(72) Inventor: PAAMAND, Rune Tore, 3050 Humlebaek (DK)
(74) Representative: Garratt, Peter Douglas

(57) **Abstract**

A medical device includes a heating element and at least one multifunctional wire coupled to the heating element. The multifunctional wire provides current for both heating the heating element and for determining the temperature of the medical device as a thermocouple. The heating element is further coupled to one or more additional wires for completing both a heating circuit and a thermocouple circuit in combination with the multifunctional wire. In one form, the multifunctional wire and a thermocouple return wire is coupled to a first end of the heating element and heating return wire is coupled to a second end of the heating element. In another form, a first multifunctional wire is coupled to the first end of the heating element, and a second multifunctional wire is coupled to the second end of the heating element.

## Description

### CROSS-REFERENCE TO RELATED APPLICATIONS

This application claims the benefit of priority to U.S. Provisional Application No. 62/312,533, filed March 24, 2016.

### FIELD

The present disclosure relates generally to medical devices. More specifically, the disclosure relates to a system and method for occluding or closing a body vessel using a medical device current to heat and/or ablate the body vessel and for determining a temperature of the medical device.

### BACKGROUND

There are numerous medical conditions when it is desired or necessary to close a body vessel, including the treatment of aneurysms, arteriovenous malformations, arteriovenous fistulas, for starving organs of oxygen and nutrients, in the treatment or containment of cancerous growths, and so on.

Several techniques are known and in use for closing or occluding such body vessels. Traditionally, vessels have been closed by means of external ligation, which generally must be carried out by an open surgery procedure, with its associated risks, inconvenience, and long patient recovery times. Other, more recent, methods aim to use an endoluminal procedure to insert into the vessel or organ one or more occlusion devices, such as a metal framed occluder, coils, pellets or the like, able to obstruct the flow of blood in the vessel.

It is also known to seek to constrict a vessel by endoluminal ablation, causing contraction of the vessel and/or coagulation of blood to form a blood clot in the vessel. Various methods can be employed to cause such ablation. One such method involves the use of a resistive heating device, where an electric current is applied to a resistive element having a relatively high resistivity. The resistive element will become hot, thereby effecting ablation.

### BRIEF SUMMARY

The invention may include any of the following embodiments in various combinations and may also include any other aspect described below in the written description or in the attached drawings. This disclosure provides a medical device and methods for conducting vessel ablation and occlusion.

In one approach, a system for providing resistive heating and temperature sensing functionality includes an elongate medical device having proximal and distal ends; a resistive heating element disposed on the distal end of the medical device, the resistive heating element having a first end and a second end; a control unit operatively coupled to the resistive heating element for applying a current to the heating element; a multifunctional wire electrically connected at one end to the first end of the heating element at a first junction point, with an opposite end of the multifunctional wire being operatively connected to the control unit; a heating return wire electrically connected at one end to the second end of the heating element at a second junction point, with an opposite end of the heating return wire being operatively connected to the control unit; a thermocouple return wire electrically connected at one end to first end of the heating element and the first junction, with an opposite end of the thermocouple return wire being operatively connected to the control unit; a heating circuit defined by the multifunctional wire, the heating element, and the heating return wire, the heating circuit operable by the control unit to provide a current to the heating element to heat the medical device; and a thermocouple circuit defined by the multifunctional wire and the thermocouple return wire, the thermocouple circuit operable by the control unit to measure a temperature of the medical device.

In another approach, a system for providing resistive heating and temperature sensing functionality includes an elongate medical device having proximal and distal ends; a resistive heating element disposed on the distal end of the medical device, the resistive heating element having a first end and a second end; a control unit operatively coupled to the resistive heating element for applying a current to the heating element; a first multifunctional wire electrically connected at one end to the first end of the heating element at a first junction point, with an opposite end of the first multifunctional wire being operatively connected to the control unit; a second multifunctional wire electrically connected at one end to the second end of the heating element at a second junction point, with an opposite end of the second multifunctional wire being operatively connected to the control unit; a heating circuit defined by the first multifunctional wire, the heating element, and the second multifunctional wire, the heating circuit operable by the control unit to provide a first current to the heating element to heat the medical device; and a thermocouple circuit defined by the first multifunctional wire, the heating element, and the second multifunctional wire, the thermocouple circuit operable by the control unit to measure a temperature of the medical device by applying a second current that is lower than the first current.

In another approach, a method of heating a medical device and detecting a temperature of a medical device includes the steps of: providing an elongate medical device having a resistive heating element disposed at a distal end thereof, the medical device further comprising a first wire coupled to the heating element and second wire coupled to the heating element, wherein the first and second wires each have a lower resistance than the resistive heating element and the material of the first wire is different than the material of the second wire; applying a first current from a control unit to the resistive heating element via the first wire and in response thereto, heating the resistive heating element via resistive heating; applying a second current from the control unit to the first wire and the second wire and detecting a voltage at the control unit; and in response to detecting the voltage at the control unit, determining a temperature of the medical device.

Various additional features and embodiments will become apparent with the following description. The present disclosure may be better understood by referencing the accompanying figures.

### BRIEF DESCRIPTION OF THE DRAWINGS

Figure 1 illustrates medical device including a resistive heating element for providing resistive heating to a body vessel;
Figures 2A-2B illustrate a distal end of the medical device;
Figure 3 illustrates a schematic representation of the medical device and a control unit including a resistive heating circuit and a thermocouple circuit;
Figure 4 illustrates an alternative medical device including a multifunctional wire, a thermocouple return wire, and heating return wire;
Figure 5 illustrates an alternative medical device including a first multifunctional wire and a second multifunctional wire;
Figure 6 illustrates an alternative medical device including a heating return wire providing support for the heating element and having a larger cross-section than the heating element; and
Figure 7 illustrates a graphical representation of a heating cycle and a temperature detection cycle.

### DETAILED DESCRIPTION

The present disclosure will now be described more fully with reference to the accompanying figures, which show various embodiments. The accompanying figures are provided for general understanding of various embodiments and method steps. However, this disclosure may be embodied in many different forms. These figures should not be construed as limiting, and they are not necessarily to scale.

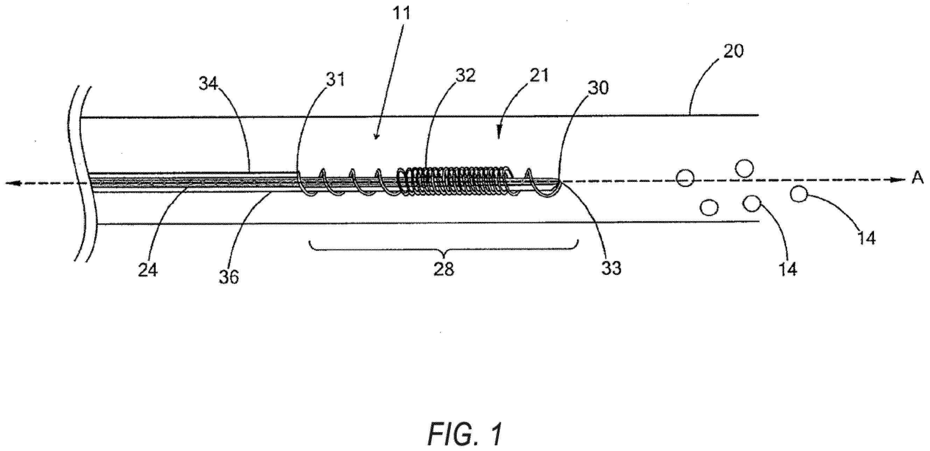

Fig. 1 depicts an environmental view of one embodiment of a medical device 11 for use in a system 10 that may be used to heat a body vessel at or adjacent a treatment site 21. The body vessel has a vessel wall 20. In this view, the device may be placed or positioned in the body vessel in a body. The device may have a support 24, or mandrel, that extends from a proximal end to a distal end 30. The support 24 may define a longitudinal axis A. It will be appreciated that the illustrated environment of the device 11 and body vessel can differ. For example, the body vessel is illustrated as being generally tubular, but the device can be used to provide ablation or occlude other shapes of body vessels or body cavities. The device 11 may have a curvature or be capable of being curved or bent similar to a common guidewire.

A resistive heating element, preferably in the form of a coil 32, may be disposed about the support 24, and the coil 32 may be electrically conductive. The electrical conductivity of the coil 32 allows for a current to be passed through the coil 32, resulting in the coil 32 becoming heated. The coil 32 may have a first end 31 being disposed proximally from the distal end 30 and the coil 32 may extend to a second end 33 being disposed at the distal end 30. The support 24 may have a distal segment 28 that supports the coil 32, or about which the coil 32 is disposed.

The coil 32 is preferably made of a material having a first predetermined resistance level. The first predetermined resistance is preferably selected to be relatively high, such that current flowing through the coil 32 will cause the coil 32 to be become easily heated, as opposed to a low resistance material, where current flowing therethrough would result in a lower amount of heating. In one approach, the coil can be made from a Nickel-Iron alloy. In another approach, the coil 32 can be made from a Nickel-Chromium alloy. It will be appreciated that other materials having a generally high resistance to accommodate resistive heating could also be used.

The coil 32 may have a first coil spacing and a second coil spacing. The second coil spacing is such that the space between the coils of the coil 32 is smaller than the thickness of the coil 32 (meaning the coils are relatively close together, or touching). The first coil spacing is such that the space between the coils is greater than the thickness of the coils (meaning the coils are generally spaced apart from each other and not touching). This described spacing applies to particular embodiments, but other spacing or lack of spacing between adjacent coils could also be used. The spacing may be consistent or may vary along the length of the device.

The distal segment 28 may have an outer diameter that distally decreases to form a distal taper (as shown in Fig. 1). Alternatively, the support 24, including its distal segment 28, may have an outer diameter being uniform from the proximal end 26 to the distal end 30. The distal taper or tapered tip may provide the advantage of making the distal end 30 easier to track within the body vessel. Such distal taper may provide flexibility to track the device. In an alternative approach, the device 11 may be made without the support 24.

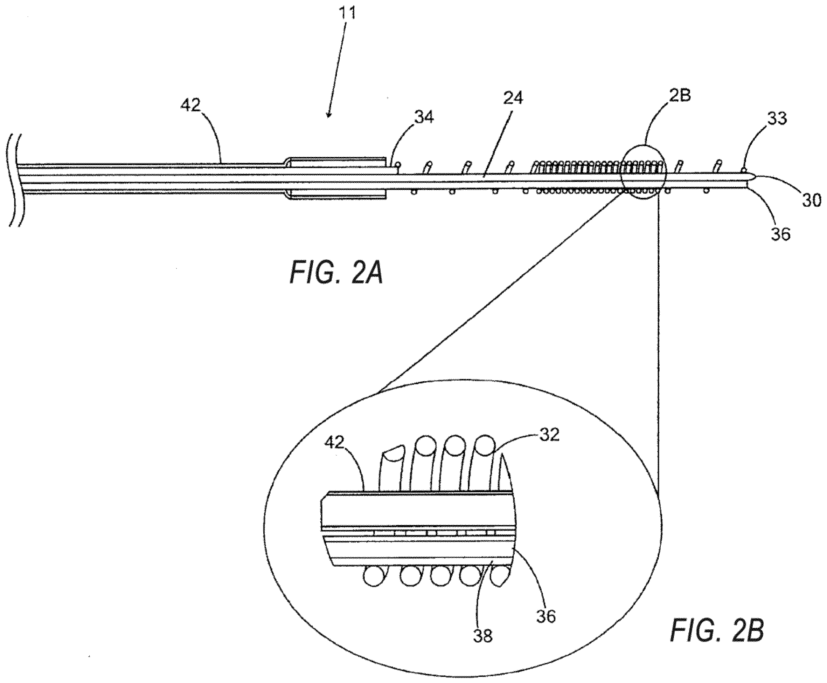

Figs. 2A-B depicts further details of the device. For example, Fig. 2A shows a cross-sectional view of the device. Fig. 2B shows a blown-up view of the device around circle 2B. In Fig. 2A, the support 24 has a uniform outer diameter from the proximal end 26 to the distal end 30. Additionally, either or both of the first and second wires (34, 36) may have an insulator 38 disposed about their outer surface to electrically insulate them from the rest of the device 11. Insulator 38 is shown disposed about the second wire 36 in Fig. 2B.

Additionally, the device 11 may have a shrink tubing disposed about the device 11. For example in Fig. 2A, the shrink tubing 42 may extend around the support 24, the first wire 34, the second wire 36, and/or any other portion of the device 11. As one advantage, the shrink tubing 42 may immobilize or bind the support 24, the first wire 34, and the second wire 36 in place so that they are immobilized relative to each other. The shrink tubing 42 may also immobilize and/or extend over a part of the coil 32 to keep the coil 32 in position as the device 11 is in use. Alternatively or additionally in Fig. 2B, the shrink tubing 42 may only be disposed about the support 24. In this configuration, the shrink tubing 42 may act to isolate or insulate the support 24 from the rest of the device 11.

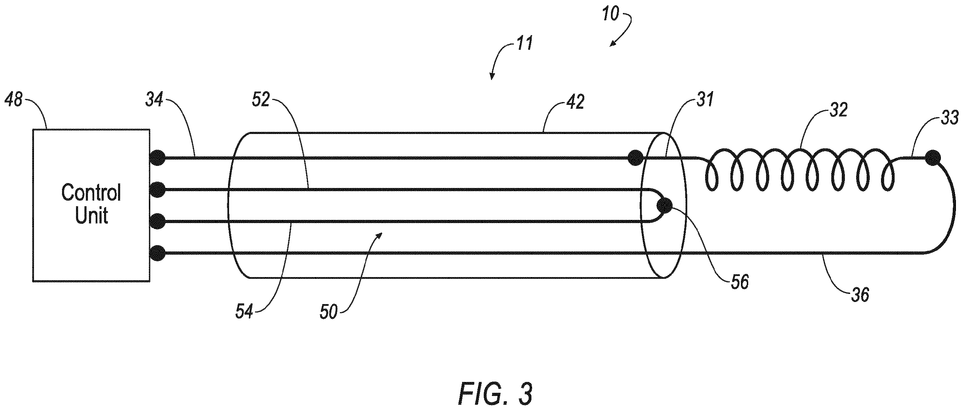

As described above, and with further reference to Figure 3 (illustrated without a support structure), the device 11 may have the first wire 34 and the second wire 36 for transferring the current through the coil 32. The first wire 34 may be electrically coupled or connected to a control unit 48. The first wire 34 may extend from the control unit 48 and along the longitudinal axis A to the first end 31 of the coil 32. The first wire 34 may be attached to the first end 31 of the coil 32 such that the connection provides an electrical and conductive coupling between the first wire 34 and the coil 32.

The second wire 36 may also be electrically coupled or connected to the control unit 48 and extend from the control unit 48, along the longitudinal axis A, to the second end 33 of the coil 32. The second wire 36 may be attached to the second end 33 such that the connection provides an electrical coupling between the second wire 36 and the coil 32. In this way, the device may form a first circuit along the path created by the control unit 48, the first wire 34, the coil 32, and the second wire 36.

In a traditional approach, the first wire 34 and the second wire 36 are each made from a low resistance material, such as copper. It will be appreciated that other low resistance materials can be used for the first and second wires. The low resistance in the first and second wires 34, 36 allows for high electrical conductivity and high current loads. Voltage/current is typically applied by the control unit 48 through the circuit formed by the first wire 34, the coil 32, and the second wire 36, where the coil 32 having a high resistance will become heated, and the first and second wires 34 and 36 will become less heated due to their lower resistances.

In a traditional approach, the system 10 can include a separate thermocouple 50 for sensing the temperature. The thermocouple 50 includes a first wire conductor 52 and a second wire conductor 54 that are coupled at a junction point 56 disposed adjacent the coil 32. The thermocouple 50 is connected to the control unit 48, which applies a current to a thermocouple circuit defined by the thermocouple 50. The measured voltage of the thermocouple circuit can be used to determine the temperature at the junction point 56 in manner known in the art.

There are various known thermocouples types, which can be selected depending on the desired application, such as temperature range or sensitivity. Some Nickel alloy thermocouples include Types E (chromel-constantan), J (iron-constantan), K (chromel-alumel), and T (copper-constantan). Other thermocouple types include Platinum/rhodium alloy thermocouples, and Tungsten/rhenium alloy thermocouples.

In one approach, the thermocouple 50 can be a Type K thermocouple, which includes chromel and alumel materials.

The above described arrangement, with the separate thermocouple 50 includes the two conductors 52, 54 made from chromel and alumel (for Type K) or two other different materials for other thermocouple types. The two conductors 52, 54 are in addition to the two wires 34, 36, thereby including a total of four wires connected to the control unit and extending along the medical device. The system 10 preferably includes the insulating sheath or shrink tubing 42 that extends over the conductors 52, 54 and wires 34, 36 to isolate them and the current flowing therethrough from adjacent body tissue. The tubing 42 preferably terminates prior to the distal end of the medical device, such that the coil 32 is exposed and capable of heating adjacent tissue when current is applied to the coil 32.

In a preferred embodiment, the number of total wires/conductors is reduced, thereby reducing the overall width of the medical device and allowing the medical device to reach narrower body vessels. In this approach, a multi-functional wire 134 is used to replace two of the four total wires, resulting in a three wire arrangement, as further described below.

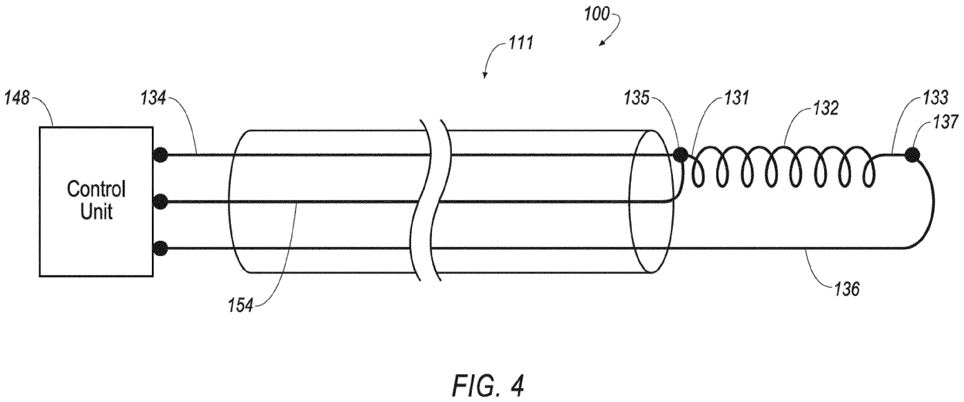

With reference to Figure 4, a medical device 111 includes a coil 132 having a first end 131 and a second end 133. In one approach, the first end 131 is disposed proximally from the second end 133, with the second end 133 being disposed at a distal end of the medical device 111. The medical device 111 is part of a system 100 that includes a control unit 148. The control unit 148 includes a processing device and a non-transitory computer readable medium having instructions stored thereon that are configured to control current and/or voltage that is applied to the medical device 111.

The medical device 111 includes the multifunctional wire 134 that extends between the control unit 148 and the coil 132. The multifunctional wire 134 is made from a material having properties with low resistivity allowing the multifunctional wire 134 to conduct a sufficient amount of current to the coil 132 to heat the coil while also not adding any substantial electrical losses or heat. Additionally, the material of the multifunctional wire 134 is selected such that it can also act as one half of a thermocouple.

The multifunctional wire 134 thereby extends between the control unit 148 and the first end 131 of the coil 132, with the multifunctional wire 134 being electrically coupled to the first end 131 of the coil 132 and a first junction point 135. The second end 133 of the coil 132 is electrically coupled to a current return wire 136 at a second junction point 137, similar to the second wire 36 described above. The current return wire 136 extends between the second junction point 137 and the control unit 148, with the current return wire 136 being operatively coupled to the control unit 148. The current return wire 136 is preferably made from a material with low resistivity and high conductivity while not adding significant electrical losses or heat. In one approach, the current return wire 136 is made from copper, which has low resistance and high conductivity. Of course, another material could be used having properties sufficient to function as described.

The multifunctional wire 134, the coil 132, and the current return wire 136 combine to define a first circuit or resistive heating circuit. The end of the current return wire 136 opposite the end that is coupled to the second junction point 137 is operatively coupled to the control unit 148, such that the control unit 148 can control the current and voltage that is applied to the resistive hearing circuit to heat the coil 132 by conducting a current therethrough.

The medical device 111 also includes a thermocouple return wire 154 that extends between the first junction point 135 and the control unit 148. The thermocouple return wire 154 is operatively coupled to the control unit 148 at one end, and the opposite end is electrically coupled to the multifunctional wire 134 at the first junction point 135 and may also be coupled to the first end 131 of the coil 132.

The thermocouple return wire 154 is preferably made from a material that is different from the material of the multifunctional wire 134, and will pair with the material used for the multifunctional wire in defining a thermocouple capable of providing a temperature that is present at the first junction point 135.

In one approach, the multifunctional wire 134 is made of copper, and the thermocouple return wire 154 is made of constantan. Thus, the thermocouple defined by the multifunctional wire 134 and the thermocouple return wire in this approach is a Type T thermocouple. Other combinations that could be used include iron-constantan and chromel-gold/iron. Of course, other combinations could also be used.

Regardless of the paired materials used to create the thermocouple, the multifunctional wire 134 and the thermocouple return wire 154 combine to define a thermocouple circuit that acts in parallel to the heating circuit. A measured voltage of the thermocouple circuit 150 can be used to determine the temperature at the first junction 135.

The junction 135, being located at the first end 131 of the coil 132 thereby provides an accurate reading of the temperature of the coil 132 when the thermocouple is used to detect the temperature at the junction 135.

In one approach, the coil 132 and the thermocouple return wire 154 could be made of the same material, and could be a unitary structure. For example, in one approach, the coil 132 and the thermocouple return wire 154 could both be made of constantan. In this approach, the multifunctional wire 134 could be coupled to the first end of the coil 132 at junction point 135 as described above. The heating circuit would be conducted through the multifunctional wire 134 and further through the coil 132 and heating return wire 136 as described above. The thermocouple circuit 150 would be completed back through the thermocouple return wire 154 via the first junction 135.

Thus, the medical device 111 includes three total wires, with the multifunctional wire 134 being part of both the heating circuit and the thermocouple circuit. The thermocouple return wire 154 completes the thermocouple circuit 150, and the heating return wire 136 and the coil 132 complete the heating circuit. The use of three wires to complete both the thermocouple circuit and the heating circuit thereby reduces the overall size of the medical device 111 relative to the medical device 11 having four wires, while still providing both heating and temperature detecting functionality.

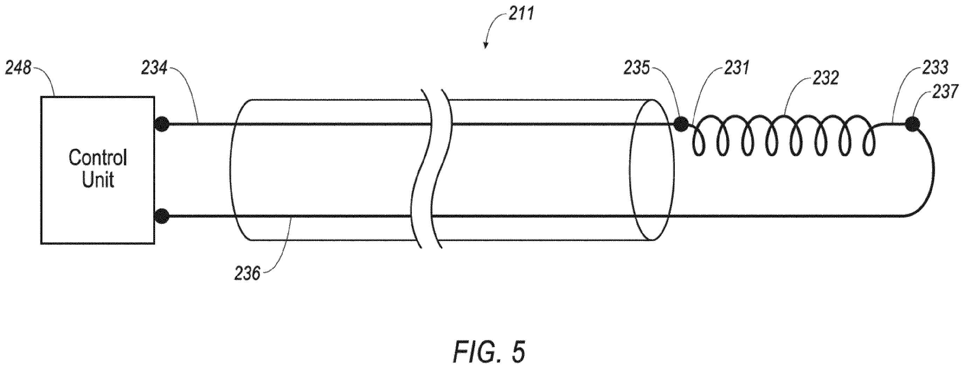

In another preferred embodiment, shown in Figure 5, a medical device 211 includes a total of two wires rather than three or four wires to provide both heating and temperature detection functionality. In this approach, a first multifunctional wire 234 extends between a control unit 248 and a heating element or coil 232. Further, a second multifunctional wire 236 extends between the coil 232 and the control unit 248.

In this approach, the first multifunctional wire 234 is electrically connected to a first end 231 of the coil 232 at a first junction point 235, and the opposite end of the first multifunctional wire 234 is operatively coupled to the control unit 248. The second multifunctional wire 236 is electrically connected to a second end 233 of the coil 232 at a second junction point 237, and the opposite end of the second multifunctional wire 236 is operatively coupled to the control unit 248.

In this approach, the first and second multifunctional wires 234 and 236 each operate to provide a current for heating the coil 232 for resistive heating. The multifunctional wires 234 and 236 are also used to provide a current for determining a temperature as a thermocouple.

Thus, a heating circuit is defined by the first multifunctional wire 234, the coil 232, and the second multifunctional wire 236, with the current being applied by the control unit 248. A thermocouple circuit is defined by the first multifunctional wire 234, the coil 232, and second multifunctional wire 236, with the current being applied by the control unit 248.

Typically, a thermocouple includes a single junction between a pair of wires having different materials. In this approach, there are two junction points 235 and 237. However, when the coil 232 is heated via resistive heating, the resulting temperature of the coil 232 is generally equal throughout the length between the two junctions 235 and 237. Thus, due to the generally equal temperature through the coil 232, the coil 232 acts as an effective single junction point, thereby allowing the thermocouple between the two multifunctional wires 234, 236. The equal temperature through the coil 232 results in no thermocouple voltage being created in the coil, allowing for a reliable temperature measurement at the location of the coil via the thermocouple.

The materials of the multifunctional wires 234 and 236 are preferably selected to have both the ability to conduct current and provide a sensitive thermocouple. The materials of the wires 234, 236 are different from each other to allow for the thermocouple functionality. Preferably, the wires 234, 236 each have low electrical resistivity to allow them to effectively carry current to the resistive element or coil 232. Further, the wires 234, 236 preferably have low thermal conductivity such that they do not sink heat from the coil 232.

In one approach, one thermocouple metal alloy combination that could be used is a Type P thermocouple using Palladium/platinum/gold alloys. Another combination that could be used is a pure noble metal thermocouple such as gold/platinum for one of the wires 234, 236 and platinum/palladium for the other of the wires 234, 236. The coil 232 can be made from a material having a sufficiently high resistivity for use with resistive heating, such as the materials described above for the coil 32.

Thus, the medical device 211 can provide both resistive heating functionality as well as temperature sensing thermocouple functionality while using only two wires, rather than the three or four described above. A two-wire device can have a reduced size relative to three and four wire devices.

As described above, the coils 32, 132, and 232 have been described as being made of a material with high resistivity to allow for resistive heating, and the wires coupled thereto that are part of the heating circuits for each have been a low resistivity material such that the current can be effectively transmitted to the coil.

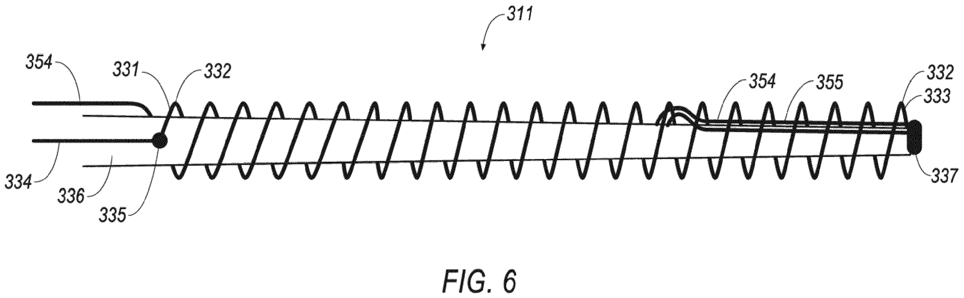

In another approach, as shown in Figure 6, the coil 332 of a medical device 311 could be made of the same material as one of the heating wires 336, with the heating wire 336 having a larger cross-section than the coil 332, thereby lowering the resistivity in the heating wire 336 relative to the coil 332, such that the coil 332 will become heated due to its high resistance relative to the heating wire 336 portion.

As shown in Figure 6, a multifunctional wire 334 is coupled to a first end 331 of heating coil 332 at a first junction point 335. A second end 333 of the heating coil 332 is coupled to heating return wire 336 at junction point 337, which has a larger cross-section. A thermocouple return wire 354 is coupled to junction point 337, and includes an insulating layer 355 to isolate it from the heating coil 332 as it extends through the heating coil 332 at the distal end thereof. The heating return wire 336 acts as a support structure for the medical device 311.

In another approach, the support structure could be made of Nitinol or stainless steel wire, which itself could be used as part of a thermocouple.

With reference to the medical devices 111, 211, 311 having at least one multifunctional wire that provides current for resistive heating as well as thermocouple measurements, it is desirable to alter the level of voltage/current applied to the heating and thermocouple circuits. In particular, it is desirable to limit electrical potentials created by current flowing to the heating element to limit corrupting the thermocouple measurements. For instance, thermocouples typically produce voltages in the order of microvolts per degree kelvin. On the other hand, voltages used for driving currents for resistive heating can be between 5-80 volts.

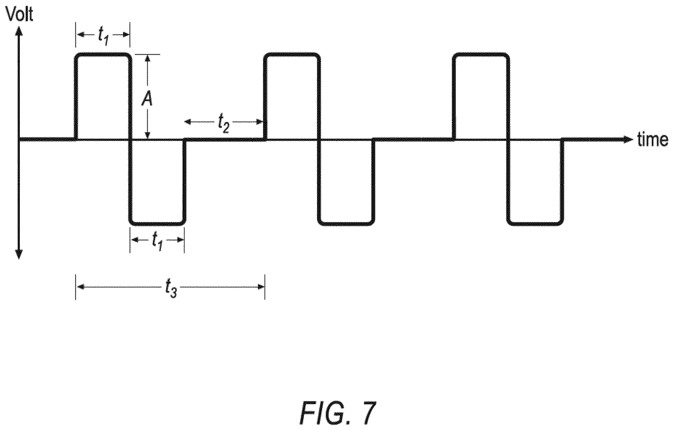

Thus, with reference to Figure 7, one method of operating the devices 111 and 211, or other devices that include a multifunctional wire, involves switching intermittently between a heating function and a temperature sensing function. Accordingly, the heating circuit and heating functionality can be activated during a first period of time, using higher voltages to effectively provide resistive heating. During this time, electrical potentials may be generated, but will not affect thermocouple measurements because the thermocouple circuit is not activated for measuring temperature.

After the first period of time, the heating circuit can be deactivated, and current/voltage can be generated at a lower magnitude during a second tie period. The coil 132 or 232 will not generally undergo substantial heating and high electrical potentials will not be generated, thereby not substantially effecting the thermocouple measurement that can occur. During this second time period, the thermocouple can produce a voltage that is used to sense the temperature at the junction point of the thermocouple.

One approach to switched or intermittent activation of the heating and thermocouple functions is illustrated in Figure 7. As illustrated, the medical device 111, 211, 311 is operated on the basis of a square-pulsed wave, providing heating current during some intervals and providing temperature sensing current during other intervals.

Figure 7 illustrates generated bipolar driving voltages over time. The overall cycle occurs over time t₃. The pulse durations occur over time t₁. The cycle frequency is 1/t₃. The temperature sensing intervals are shown as time t₂. The duty cycle is 2*t₁/t₃.

The above described time periods of alternating resistive heating and thermocouple operation is merely exemplary. In one approach, the time period t3 could be 1-10 microseconds and pulse frequency of higher than 100kHz can be used. An advantage of a pulse frequency higher than 100kHz is the absence of nerve stimulation and increased safety. It will be appreciated that different periods of time or patterns of heating and temperature detection could also be used.

The above description relates to the use of one or more multifunctional wires for providing resistive heating to a resistive heating element along with thermocouple capabilities. The above described embodiments could also be used in other applications where temperature is monitored within the body, such as catheter based RF heating procedures. The above described embodiments could also be used in applications where the medical device is not in the body, for example demonstrating, training or testing applications.

It should be understood that the foregoing relates to exemplary embodiments of the disclosure and that modifications may be made without departing from the spirit and scope of the disclosure as set forth in the following claims. While the disclosure has been described with respect to certain embodiments it will be appreciated that modifications and changes may be made by those skilled in the art without departing from the spirit of the disclosure.

## Claims

1. A system for providing resistive heating and temperature sensing functionality, the system comprising:
an elongate medical device having proximal and distal ends;
a resistive heating element disposed on the distal end of the medical device, the resistive heating element having a first end and a second end and preferably being a coil;
a control unit operatively coupled to the resistive heating element for applying a current to the heating element;
a multifunctional wire electrically connected at one end to the first end of the heating element at a first junction point, with an opposite end of the multifunctional wire being operatively connected to the control unit;
a heating return wire electrically connected at one end to the second end of the heating element at a second junction point, with an opposite end of the heating return wire being operatively connected to the control unit;
a thermocouple return wire electrically connected at one end to first end of the heating element and the first junction, with an opposite end of the thermocouple return wire being operatively connected to the control unit;
a heating circuit defined by the multifunctional wire, the heating element, and the heating return wire, the heating circuit operable by the control unit to provide a current to the heating element to heat the medical device; and
a thermocouple circuit defined by the multifunctional wire and the thermocouple return wire, the thermocouple circuit operable by the control unit to measure a temperature of the medical device.

2. The system of claim 1, wherein the multifunctional wire and the thermocouple return wire are made of different materials for providing thermocouple functionality.

3. The system of claim 1 or claim 2, wherein the heating element has a resistance that is higher than the resistance of the multifunctional wire and the heating return wire.

4. The system of any one of the preceding claims, wherein the thermocouple return wire and the coil are a unitary structure made of the same material.

5. The system of any one of the preceding claims, wherein the multifunctional wire is made of copper and the thermocouple return wire is made of constantan, wherein the heating return wire is preferably made of copper, wherein the heating element is preferably made of a material having a resistance greater than copper and wherein the heating element is preferably made of constantan.

6. The system of any one of the preceding claims, wherein the thermocouple return wire and the heating element are made of different materials.

7. A system for providing resistive heating and temperature sensing functionality, the system comprising:
an elongate medical device having proximal and distal ends;
a resistive heating element disposed on the distal end of the medical device, the resistive heating element having a first end and a second end;
a control unit operatively coupled to the resistive heating element for applying a current to the heating element;
a first multifunctional wire electrically connected at one end to the first end of the heating element at a first junction point, with an opposite end of the first multifunctional wire being operatively connected to the control unit;
a second multifunctional wire electrically connected at one end to the second end of the heating element at a second junction point, with an opposite end of the second multifunctional wire being operatively connected to the control unit;
a heating circuit defined by the first multifunctional wire, the heating element, and the second multifunctional wire, the heating circuit operable by the control unit to provide a first current to the heating element to heat the medical device; and
a thermocouple circuit defined by the first multifunctional wire, the heating element, and the second multifunctional wire, the thermocouple circuit operable by the control unit to measure a temperature of the medical device by applying a second current that is lower than the first current.

8. The system of claim 7, wherein the first multifunctional wire and the second multifunctional wire are made of different materials to provide thermocouple functionality.

9. The system of claim 7 or claim 8, wherein the heating element provides a generally equally temperature at both the first junction and the second junction in response to resistive heating.

10. The system of any one of claim 7 to claim 9, wherein the material of the heating element is different than the materials of the first and second multifunctional wires, and the heating element has a higher resistance than the first and second multifunctional wires.

11. The system of any one of claim 7 to claim 10, wherein one of the first and second multifunctional wires is made of gold/platinum and the other of the first and second multifunctional wires is made of platinum/palladium.

12. A method of heating a medical device and detecting a temperature of a medical device, the method comprising the steps of:
providing an elongate medical device having a resistive heating element disposed at a distal end thereof, the medical device further comprising a first wire coupled to the heating element and second wire coupled to the heating element, wherein the first and second wires each have a lower resistance than the resistive heating element and the material of the first wire is different than the material of the second wire;
applying a first current from a control unit to the resistive heating element via the first wire and in response thereto, heating the resistive heating element via resistive heating;
applying a second current from the control unit to the first wire and the second wire and detecting a voltage at the control unit; and
in response to detecting the voltage at the control unit, determining a temperature of the medical device.

13. The method of claim 12, wherein the first wire is attached to a first end of the heating element at a first junction point and the second wire is attached to a second end of the heating element at a second junction point, and the heating element is heated generally equally at both the first and the second junction points via the resistive heating.

14. The method of claim 12 or claim 13, wherein the first wire is attached to a first end of the heating element at a first junction point, the second wire is attached to the first wire at the first junction point and is further attached to the heating element at the first junction point, and the medical device further comprises a third wire attached to a second end of the coil at a second junction point, and the first current for resistive heating is applied to a circuit defined by the first wire, the coil, and the third wire, preferably wherein the second wire and the heating element are made of the same material and are a unitary structure

15. The method of any one of claim 12 to claim 14, wherein the first current is applied for heating the heating element during a first period of time defining a duty cycle, and the second current is applied for determining the temperature during a second period of time following the first period of time.
